**Europäisches Patentamt**

**European Patent Office**

**Office européen des brevets**

(19)

(11) Veröffentlichungsnummer: **0 122 604**
**B1**

(12) # EUROPÄISCHE PATENTSCHRIFT

(45) Veröffentlichungstag der Patentschrift:
**20.07.88**

(51) Int. Cl.⁴: **A 61 M 1/34**

(21) Anmeldenummer: **84104120.5**

(22) Anmeldetag: **12.04.84**

(54) Einrichtung zum Regeln der Ultrafiltrationsrate.

(30) Priorität: **13.04.83 DE 3313421**

(43) Veröffentlichungstag der Anmeldung:
**24.10.84 Patentblatt 84/43**

(45) Bekanntmachung des Hinweises auf die Patenterteilung:
**20.07.88 Patentblatt 88/29**

(84) Benannte Vertragsstaaten:
**DE FR GB IT SE**

(56) Entgegenhaltungen:
**EP-A-0 029 793**
**EP-A-0 042 939**
**EP-A-0 062 913**
**EP-A-0 089 003**
**DE-A-2 644 062**
**DE-A-3 132 790**
**GB-A-2 003 274**
**US-A-3 619 423**
**US-A-4 370 983**

(73) Patentinhaber: **Fresenius AG, Gluckensteinweg 5,
D-6380 Bad Homburg v.d.H. (DE)**

(72) Erfinder: **Polaschegg, Hans- Dietrich, Dr.,
Grünwiesenweg 9, D-6370 Oberursel (DE)**

(74) Vertreter: **Luderschmidt, Wolfgang, Dr. Dipl-
Chem., Görtz, Dr. Fuchs, Dr. Luderschmidt
Patentanwälte Sonnenberger Strasse 100
Postfach 26 26, D-6200 Wiesbaden (DE)**

EP 0 122 604 B1

## Beschreibung

Die Erfindung betrifft eine Vorrichtung zur extrakorporalen Regelung der Ultrafiltrationsrate gemäß dem nichtkennzeichnenden Teil des Anspruchs 1.

Sie betrifft insbesondere eine Einrichtung zum Regeln der Ultrafiltrationsrate bei Hämodialysevorrichtungen, die einen Dialysator mit einem Blutkreislauf und einem Dialysierflüssigkeitskreislauf aufweisen, wobei in dem Dialysierflüssigkeitskreislauf eine Einrichtung zur Entziehung von Ultrafiltrat vorgesehen ist.

Bei der Hämodialyse wird neben den Stoffwechselprodukten, wie Harnstoff, auch Wasser dem Blut des Patienten in Form von Ultrafiltrat entzogen. Dabei hat es sich gezeigt, daß es äußerst wichtig ist, diesen Flüssigkeitsentzug exakt zu steuern, um die beim Patienten auftretenden Entzugsprobleme möglichst gering zu halten bzw. zu vermeiden. Hierzu ist insbesondere die Ultrafiltrationssteuerung von herausragender Bedeutung. Dabei ist zu unterscheiden zwischen offenen und geschlossenen Systemen, die in Single-Pass-Vorrichtungen verwirklicht sind.

In der DE-A-2 644 062 ist eine Vorrichtung zur automatischen Regelung eines Hämodialysegerätes mit offenem Kreislauf beschrieben, bei dem im Dialysierflüssigkeitskreislauf stromauf des Dialysators ein Magnetventil und stromab des Dialysators eine konstant fördernde Pumpe angeordnet sind. Das Magnetventil ist mit einer Regeleinheit verbunden, die das Megnetventil derart steuert, daß eine vorgewählte Menge Wasser dem Patienten während der Dialyse kontinuierlich entzogen wird. Da sich jedoch der Strömungswiderstand der Dialysatormembran während der Dialyse, beispielsweise durch Verstopfung und dergl., ändert, muß regelmäßig die gesamte Anordnung stillgelegt und der Strömungswiderstand bestimmt werden, was einerseits zeitaufwendig und vor allen Dingen vorrichtungstechnisch problematisch ist.

Dies ist zum einen darauf zurückzuführen, daß die Pumpe im Dialysierflüssigkeitskreislauf das Ultrafiltrat abfördert, das gegenüber der Dialysierflüssigkeitsmenge gering bleibt (höchstens 1 %) und somit nicht direkt bestimmt werden kann, so daß evtl. auftretende Fehler nicht unmittelbar erkannt werden können, und zum anderen aber auch darauf, daß das Ultrafiltrat selbst erst dann gemessen wird, wenn es den Blutkreislauf bereits verlassen hat.

Die Bestimmung des Ultrafiltrats auf der Dialysierflüssigkeitsseite hat also den Nachteil, daß beispielsweise ein langsamer Blutverlust in die Umgebung (z. B. etwa 10 ml/min) nicht erkannt wird, da der Blutverlust noch nicht ausreicht, um den Transmembrandruck wirksam zu verändern und somit eine auf Druck ansprechende Sicherheitseinrichtung zu betätigen.

Dasselbe gilt auch für Entnahmen von Blutproben aus dem Blutkreislauf, die zu einer Verfälschung der Ultrafiltratbestimmung führen. In ähnlicher Weise kann eine Infusion einer Ersatzflüssgkeit in die Blutleitung zu einer nicht meßbaren Veränderung der Ultrafiltratbilanz führen.

Aus der DE-A-2 259 787 ist eine Hämodialysevorrichtung bekannt, bei der Flüssigkeit aus einem geschlossenen Dialysierflüssigkeitskreislauf entzogen wird. Diese Vorrichtung weist jedoch den Nachteil auf, daß die Dialysierflussigkeit rezirkuliert wird, so daß gegenüber dem Single-Pass-Verfahren die Effektivität der Dialyse aufgrund eines sinkenden Konzentrationsgradienten mit der Zeit vermindert wird.

Ein Single-Pass-Gerät mit einem geschlossenen System ist aus der DE-A-2 838 414 bekannt, bei dem dem Dialysator bilanziert Dialysierflüssigkeit zugeführt wird, die von und zu einem Bilanzkammersystem umgepumpt wird. Dem abgeschlossenen System wird wiederum mittels einer Ultrafiltrationspumpe eine bestimmte Menge Flüssigkeit entzogen, die durch eine entsprechende, tatsächlich ultrafiltrierte und somit dem Blut entzogene Flüssigkeitsmenge im geschlossenen System ersetzt wird.

Eine weitere Vorrichtung, die beispielsweise aus der DE-A-3 020 756 bekannt ist, bestimmt die Ultrafiltrationsrate aus der Differenz der zufließenden und der abfließenden Dialysierflüssigkeitsmenge mit Hilfe von Durchflußmessern. Dabei kann der Transmembrandruck so eingestellt werden, daß die Ultrafiltrationsrate einer vorgegebenen Rate entspricht.

Diese bekannten Hämodialysegeräte weisen zusätzlich den Nachteil auf, daß Luftblasen in der Dialysierflüssigkeit das exakte Arbeiten der Vorrichtung stören, da hierdurch die Ultrafiltrationsmenge verfälscht wird. Um dies zu vermeiden, sind hierzu spezielle Einrichtungen, beispielsweise Luftabscheider, vorgesehen.

Darüberhinaus sind diese Vorrichtungen sehr aufwendig ausgelegt, da die abzuziehende Ultrafiltrationsmenge von etwa 2 Liter einem Verbrauch an Dialysierflüssigkeit in der Höhe von etwa 120 - 240 Liter gegenübersteht, d.h. daß die Ultrafiltrationsmenge von einer relativ großen Menge Dialysierflüssigkeit überlagert wird. Da somit der Anteil des Ultrafiltrats an der Dialysierflussigkeit nur etwa 1 - 2 % beträgt, muß die gesamte Vorrichtung sehr genau beim Bilanzieren und Ultrafiltrieren arbeiten, um den Patienten nicht in Gefahr zu bringen.

Diese bekannten Systeme stellen somit die Ultrafiltration auf der Seite der Dialysierflüssigkeit ein, ohne daß auf die Bedingungen des Blutkreislaufs direkt abgestellt wird, ein.

Aus der DE-A-2 607 022 ist eine Hämofiltrationsvorrichtung bekannt, mit der die Substitutionslösung bilanziert zugeführt wird, wobei die die Substitutionslösung fördernde Pumpe mit Hilfe einer Ultraschalldurchflußmeßanordnung geregelt wird. Ein derartiger Ultraschalldurchflußmesser weist jedoch erhebliche Ungenauigkeiten auf, da er nur ein bestimmtes Volumenteil der in einer Leitung strömenden Flüssigkeit, nicht jedoch die gesamte Flüssigkeitsmenge bestimmen kann. Demzufolge führen eine Änderung im Strömungsprofil der Flüssigkeit oder die im Blut übliche pulsatile Strömung zu Verfälschungen, was zur Folge hat, daß ein derartiges System nicht genau arbeiten kann. im übrigen kann ein derartiger Durchflußmesser nicht genau justiert werden, was jedoch zwingend notwendig ist.

Die Ultrafiltration gemäß dieser Druckschrift weist den Nachteil auf, daß die Ultrafiltrationsrate infolge Verstopfung des Filters sowie Veränderungen der Blutdruckwerte im Laufe der Behandlung abgeändert werden kann. Demzufolge wird mit dieser Vorrichtung lediglich eine Substitutionslösung nachgeführt, ohne daß jedoch eine gesteuerte Ultrafiltration, insbesondere eine Ultrafiltration möglich wäre, die von einem wechselnden Blutdruck oder von Veränderungen der Membran (Verstopfen der Membran) unabhängig wäre.

Darüberhinaus weisen diese bekannten Vorrichtungen jeweils ein Luftdetektorsystem sowie Systeme zur Erkennung von Blutverlusten auf, welche unabhängig von den Steuerungen der Ultrafiltration bzw. der Substitutionslösungszugabe angeordnet sein müssen. Hierdurch werden natürlich die Vorrichtungen wiederum verteuert, ganz abgesehen davon, daß sich hierdurch weitere Fehlermöglichkeiten ergeben.

Weiterhin ist festzustellen, daß die bekannten Ultrafiltrationsmeßeinrichtungen bzw. Substitutionslösungssteuerungseinrichtungen generell den Nachteil aufweisen, daß sie stets auf ein spezielles Verfahren ausgerichtet sind und nicht miteinander kombiniert werden können.

Eine Vorrichtung gemäß dem nichtkennzeichnenden Teil der Anspruchs 1 ist beispielsweise aus der US-A-3 908 653 bekannt. Eine solche Vorrichtung unterscheidet sich von der üblicherweise angewandten Zwei-Nadel-Methode dadurch, daß alternierend die Vorrichtung zuerst in den Blutzuflußbetrieb und hierauf in den Blutabflußbetrieb geschaltet wird, während bei der Zwei-Nadel-Methode kontinuierlich Blut in einem zwei Nadeln aufweisenden Kreislauf geführt wird.

Insofern unterscheidet sich im Prinzip eine Single-Needle-Dialysevorrichtung in ihrer gesamten Steuerung und ihrem Ultrafiltrationsverhalten von der Zwei-Nadel-Vorrichtung, bei der üblicherweise der zur Ultrafiltration notwendige Druck als Unterdruck auf der Dialysierflüssigkeitsseite aufgebaut wird und somit die zur Erzielung dieses Unterdrucks notwendigen Unterdruck- und Steuereinheiten ebenfalls auf der Dialysierflüssigkeitsseite vorliegen.

In der US-A-4 370 983 ist ein computergesteuertes Hämodialysesystem nach der Zwei-Nadel-Methode beschrieben, bei dem im Blutkreislauf Durchflußmesser angeordnet sind, mit denen die Ultrafiltrationsrate mittels Differenzbildung von Blutdurchflußmeßwerten vor und hinter dem Dialysator bestimmt werden kann.

Die Regelung der Ultrafiltrationsrate wird jedoch auf der Basis von Druckmeßwerten im Blut- und Dialysierflüssigkeitsweg vorgenommen.

Die EP-A-0 029 793 beschreibt eine Hämodialysevorrichtung, bei der Elektroden in den Körper eines Patienten und Elektroden in den extrakorporalen Kreislauf unmittelbar eingesetzt werden sollen. Eine derartige Meßanordnung ist jedoch zu ungenau, da der Körper viel zu langsam auf Unterschiede in der sich durch die Ultrafiltration ergebenden Flüssigkeitsveränderung anspricht. Insofern ist eine derartige Anordnung zur Anzeige geringer Flüssigkeitsunterschiede nicht geeignet. Darüberhinaus ist weder die Regelung einer Ultrafiltrationspumpe noch der Einsatz bei einem Single-Needle-Gerät vorgeschlagen worden.

Demgegenüber liegt also der Erfindung die Aufgabe zugrunde, die aus dem nichtkennzeichnenden Teil des Anspruchs 1 bekannte Single-Needle-Vorrichtung so weiter zu bilden, daß die Ultrafiltration exakt mit einfachen Mitteln eingestellt werden kann.

Die Lösung dieser Aufgabe erfolgt durch die Merkmale des kennzeichnenden Teils des Anspruchs 1.

Mit der erfindungsgemäßen Single-Needle-Vorrichtung ist die Möglichkeit eröffnet worden, eine exakte Ultrafiltration durchzuführen, ohne daß auf der Dialysierflüssigkeitsseite im gegenüber der Atmosphäre abgeschlossenen Kreislauf arbeitende Bilanziersysteme notwendig wären. Somit kann der Dialysierflüssigkeitsweg im gegenüber der Atmosphäre offenen System betrieben werden, d.h., es kann eine die Dialysierflüssigkeit im Durchfluß fördernde Pumpe eingesetzt werden, mit der zusätzlich ein Unterdruck im Dialysierflüssigkeitsweg erzeugt werden kann. Ein derartiges System ist erheblich einfacher in seinem technischen Aufbau und seiner technischen Steuerung als ein Bilanzierungssystem mit Bilanzkammern, das exakt elektronisch gesteuert werden muß.

Die erfindungsgemäße Vorrichtung weist zunächst den Vorteil auf, daß die Ultrafiltrationsrate unmittelbar anhand der gemessenen Blutparameter bestimmt werden kann. Bisher erfolgte nämlich die Entziehung von Ultrafiltrat ohne direkte Messung der Verhältnisse im Blut, so daß also eine direkte Kontrolle der Ultrafiltration anhand der im Blut vorliegenden Verhältnisse nicht möglich war.

Erfindungsgemäß wird nunmehr die dem Blut unmittelbar entzogene Ultrafiltrationsmenge gemessen und mit einem vorgegebenen Wert verglichen. Bei Abweichungen wird die im Dialysierflüssigkeitskreislauf vorgesehene Ultrafiltratpumpe entsprechend geregelt.

Durch die Bereitstellung der erfindungsmäßen Vorrichtung entfallen die derzeit am häufigsten verwendeten Dislysierflussigkeitsbilanzierungsysteme die eine genaue Ultrafiltratmessung und -regelung ermöglicht haben. Gegenüber diesen kostspieligen und technisch aufwendigen Bilanzierungssystemen ist das erfindungsgemäße Ultrafiltratregelungssystem erheblich einfacher ausgestaltet, was sich auch in den Kosten ausdrückt, völlig abgesehen davon, daß die Ultraf iltratrate nunmehr direkt regelbar und nicht von konstante Mengen abziehenden Pumpen abhängig ist.

Diese unmittelbare Regulierungsmöglichkeit ist insbesondere deshalb von wesentlicher Bedeutung, da Apparatefehler infolge Rückkoppelung schnell erkannt werden können und zur sofortigen Abschaltung der Vorrichtung führen.

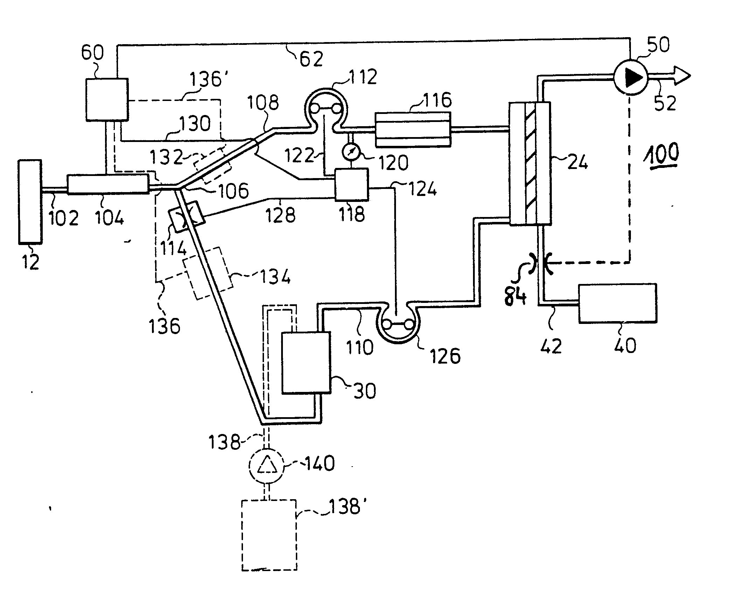

Ein Ausführungsbeispiel ist nachstehend unter Bezugnahme auf die Zeichnung erläutert:

Es zeigt die einzige Figur eine schematische Anordnung einer Hämodialysevorrichtung für die Single-Needle-Behandlung.

Ein Dialysator 24 weist einen Dialysierflüssigkeitskreislauf auf. Dieser besteht aus einem Dialysierflüssigkeitsreservoir 40, dessen Ausgang über ein Schlauchsfück 42 mit dem Dialysierflüssigkeitseingang des Dialysators 24 verbunden ist.

Der Dialysierflüssigkeitsausgang des Dialysators ist über ein weiteres Schlauchstück mit einer Pumpe 50 verbunden, die vorzugsweise als Ultrafiltratpumpe eingesetzt wird. An diese Pumpe 50 schließt sich ein Schlauchstück 52 an, das mit dem nicht gezeigten Abfluß verbunden ist. Die Pumpe 50 wirkt mit einer am Schlauchstück 42 vorgesehenen Drossel 84 zusammen, wobei der Ultrafiltration benötigte Druch erzeugt wird.

In der Figur ist eine Ausführungsform einer Unipunkturoder Single-Needle-Vorrichtung 100 gezeigt. Dementsprechend weist der Patient 12 lediglich einen Schlauchanschluß 102 auf, der in einen Durchflußmesser 104 eingelegt ist. Dieser Schlauch 102 verzweigt sich bei 106, wobei bekanntermaßen ein zum Dialysator 24 hinführendes Schlauchstück 108 und ein vom Dialysator 24 wegführendes Schlauchstück 110 vorgesehen sind.

Das Schlauchstück 108 weist eine Blutpumpe 112 auf, die das Blut vom Patienten durch den Durchflußmesser und die Leitung 108 zum Dialysator beim Pumpbetrieb fördert. Dabei ist das Schlauchstück 110 durch eine Schlauchklemme 114 verschlossen. Um ein ausreichendes Blutvolumen, beispielsweise ca. 30 - 50 ml für die Dialysebehandlung zur Verfügung zu stellen, ist das Schlauchstück 108 an einen elastischen Speicher 116 angeschlossen, in dem das Blut beim Pumpbetrieb zwischengelagert wird und der das Blut nach dem Einstellen des Pumpens infolge der auf das gespeicherte Blut einwirkenden elastischen Kräfte zum Dialysator 24 und in die Ablaßleitung 110 drückt. Dabei wirkt die Schlauchpumpe 112 wie eine Klemme.

Die Umschaltung erfolgt mit einer Steuereinheit 118, die auch mit einem Drucksensor 120 verbunden ist, der an das Schlauchstück 108 zwischen der Pumpe 112 und dem Dialysator 24 angeschlossen ist.

Die Steuereinheit 118 ist über die Leitung 122 mit der Pumpe 112, über die Leitung 124 mit der Pumpe 126 und über die Leitung 128 mit der Schlauchklemme 114 in Verbindung. Die Steuerung erfolgt derart, daß bei laufender Pumpe 112 die Pumpe 126 steht und die Klemme 114 geschlossen ist. Erst wenn der an der Steuereinheit 118 vorwählbare Druck durch den Drucksensor 120 angezeigt wird, schaltet die Steuereinheit 118 um, so daß die Pumpe 112 abgestellt und die Pumpe 126 in Betrieb genommen wird. Zugleich wird auch die Klemme 114 geöffnet.

Die Umschaltung erfolgt dann wieder, wenn ein unterer, auf der Steuereinheit 118 vorgewählter Druck unterschritten wird, wodurch die Pumpe 126 und die Klemme 114 abgestellt und die Pumpe 112 wieder eingeschaltet wird.

Infolge der zwangsläufig diskontinuierlichen Steuerung dieser Vorrichtigung 100 fließt durch den Durchflußmesser 104 im Pumpbetrieb ungereinigtes Blut, während beim Rückpumpen gereinigtes Blut durch den Durchflußmesser befördert wird. Dies hat natürlich unterschiedliche Meßparameter zur Folge, die vom Durchflußmesser an den Ultrafiltratregler 60, der über die Leitung 62 mit der Pumpe 60 verbunden ist, abgegeben und dort gespeichert werden.

In einer ersten bevorzugten Ausführungsform wird das hierdurch beim Durchflußmesser 104 erzeugte Wechselspannungssignal zur Steuerung des Ultrafiltratreglers herangezogen, während gemäß einer zweiten bevorzugten Ausführungsform das Umschaltsignal der Steuereinheit 118 auch an den Ultrafiltratregler 60 über die Leitung 130 angelegt wird.

Gemäß einer weiteren Ausführungsform kann - wie bereits vorstehend erläutert - auch die Ultrafiltration mit positivem Blutdruck anstelle eines negativen Dialysierflüssigkeitsdrucks erfolgen. In diesem Fall steuert der Ultrafiltrationsregler 60 die Umschaltpunke der Steuereinheit 118 so, daß sich ein zeitlich gemittelter Transmembrandruck einstellt, der die gewünschte Ultrafiltration bewirkt.

Da es sich um einen diskontinuierlichen Betrieb handelt, werden die jeweils erzeugten Signale zeitlich gespeichert und anschließend die zur Regelung der Pumpe 50 erforderliche Differenz gebildet. Der Ultrafiltratregler 60 steuert nun die Pumpe 50 entsprechend der gewünschten Ultrafiltrationsrate.

Soll beispielsweise keine Ultrafiltration stattfinden, so wird die Pumpe 50 so gesteuert, daß der Mittelwert der vom Durchflußmesser 104 ermittelten Meßsignale Null ist.

Gemäß einer weiteren vorteilhaften Ausführungsform können natürlich auch anstelle eines einzigen Durchflußmessers 104 zwei Durchflußmesser 132 und 134 vorgesehen sein, die sich am Schlauchstück 108 bzw. 110 befinden. In der Figur sind diese Durchflußmesser strichliert eingezeichnet. Sie sind über ebenfalls strichliert eingezeichnete Leitungen 136 und l36' mit dem Ultrafiltratregler 60 in Verbindung. Auch in diesem Fall wird die Differenz des zeitlichen Mittels der ermittelten Werte gebildet. Vorteilhafterweise wird man die Werte nacheinander ermitteln und dann die Differenz bilden. Zur Steuerung für diese Meßfolge wird wiederum die Steuereinheit 118 herangezogen, die über die Leitung 130 mit dem Ultrafiltratregler 60 verbunden ist.

Die Hämofiltration und die Hämodiafiltration nach dem Single-Needle-Verfahren wird unter Anwendung der in der Figur gezeigten Vorrichtung dadurch durchgeführt, daß eine Tropfkammer 30, die in das venöse Schlauchstück 110 eingeschaltet ist, mit einem ebenfalls strichliert eingezeichneten Leitungsstück 138 verbunden ist, das wiederum mit einem Substitutionsflüssigkeitsreservoir 138' und einer Pumpe 140 verbunden ist. Anstelle des Reservoirs 138' kann natürlich auch eine Sterilfiltrationsanordnung eingesetzt werden, bei der Dialysierflüssigkeit aus der Dialysierflüssigkeitsquelle 40 durch ein steril und pyrogenfrei wirkendes Filter zur Tropfkammer 30 gepumpt wird.

Als Durchflußmesser kommen im Prinzip sämtliche bekannten Durchflußmesser in Frage. Da hier jedoch ein Blutfluß gemessen werden soll, sind jene Durchflußmesser bevorzugt, die praktisch berührungslos arbeiten, d.h. Ultraschalldurchflußmesser, elektromagnetische Durchflußmesser sowie thermische Durchflußmesser. Besonders bevorzugt sind elektromagnetische Durchflußmesser.

Üblicherweise würde ein einzelner Durchflußmesser nicht die gewünschte Genauigkeit aufweisen und somit nicht zur Ultrafiltrationsbestimmung geeignet sein. So weist beispielsweise ein handelsüblicher elektromagnetischer Durchflußmesser eine Genauigkeit von 2 % auf. Da es erfindungsgemäß jedoch darauf ankommt, eine Differenz von Volumina genau zu messen, ist weniger die absolute Genauigkeit als vielmehr die relative Genauigkeit dieser Durchflußmesser ausschlaggebend. Bei entsprechender Auswahl des Schlauchmaterials kann der Fehler bei $10^{-3}$ - $10^{-4}$ gehalten werden, was für die Regelung der Ultrafiltrationsrate ausreicht.

In den bekannten extrakorporalen Blutkreisläufen, die mit einer Blutpumpe arbeiten, werden Luftdetektoren verwendet. Da in diesen Systemen mit dem Einsatz einer Blutpumpe gewöhnlich an einer Stelle des Systems ein Unterdruck auftaucht, ist es möglich, daß Luft angesaugt wird und über die Vene zurück in den Körper gepumpt wird, was zu Luftembolien führen kann. Um dies zu vermeiden, sind entsprechende Schutzeinrichtungen in Form von Ultraschallluftdetektoren üblich. Diese Luftdetektoren können jedoch entfallen, da Durchflußmesser, insbesondere elektromagnetische Durchflußmesser, äußerst empfindlich auf Luftblasen reagieren und somit als Schutzsystem gegen die Förderung von Luft in den Körper des Patienten eingesetzt werden können.

Wie bereits vorstehend erläutert, werden vorzugsweise elektromagnetische Durchflußmesser eingesetzt, bei denen zumindest im Bereich der Meßstrecke ein hinreichend gleichmäßiges elektromagnetisches Feld herrscht. Hierzu werden in die Schläuche jeweils zwei Elektroden eingesetzt, an denen das elektromagnetische Feld angelegt wird. Diesen wird jeweils eine Spannung entnommen, die proportional der Differenz der Durchflußgeschwindigkeiten ist, die in den beiden Meßröhren oder Schlauchstücken gleichen Durchmessers herrscht.

Demzufolge werden - wie bereits vorstehend erläutert vorzugsweise sorgfältig ausgesuchte Schläuche gleichen Durchmessers gewählt, die bereits werksseitig implantierte Elektroden aufweisen.

Die erfindungsgemäße Vorrichtung eignet sich nicht nur für die Dialyse; sie kann auch in anderen Bereichen zur Behandlung von Patienten, beispielsweise in einer Herz-Lungen-Maschine, eingesetzt werden.

Als zu den Differenzdurchflußmessern gehöriges Ultrafiltrationssteuerungssystem eignet sich auch ein solches System, das mit Sensoren zur kontinuierlichen Bestimmung des Hämatokrit im Blutzufluß und im Blutabfluß der Dialysevorrichtung arbeitet. Bekanntlich läßt sich über den Hämatokritwert der Plasmabestandteil bzw. bei Differenzbildung die ultrafiltrierte Flüssigkeitsmenge bestimmen. So können beispielsweise die Durchflußmesser auch zur kontinuierlichen Bestimmung des Hämatokrits herangezogen werden, wobei vorteilhafterweise hiermit die elektrische Leitfähigkeit von Vollblut bestimmt wird. Ist der absolute Hämatokritwert näherungsweise bekannt, so genügt zur Bestimmung der Differenz jeweils ein als Durchflußmesser ausgebildeter Leitfähigkeitssensor im Zufluß bzw. im Abfluß, wobei diese Leitfähigkeitssensoren vorteilhafterweise temperaturkompensiert sind.

Weiterhin kann der Hämatokritwert auch mit optischen Methoden gemessen werden. Dies kann beispielsweise über Lichtabsorption oder über Lichtstreuung oder über eine Kombination dieser beiden Verfahren erfolgen. Auch die Vorrichtungen, die mit diesen Verfahren arbeiten, werden zu den Durchflußmessern zugehörig erachtet und geben quantitativ die Ultrafiltrationsrate bei Erhöhung des Hämatokritwerts im Blutrückfluß zum Körper relativ zum Blutzufluß vom Körper wieder, da durch die Ultrafiltration nur Wasser, nicht aber Blutkörperchen oder Eiweiß entzogen werden.

Darüber hinaus kann der Gradient der Hämatokrit-Veränderung im Zufluß, wie von Stiller & Mann in Biomedizinischer Technik, Bd. 25 (1980), S. 286 beschrieben, gleichfalls zur Ultrafiltrationskontrolle herangezogen werden.

Darüber hinaus können zur Absolutbestimmung des Hämatokrit auch optische Ausführungen mit kalibrierten Sensoren dienen.

Derartige vorstehend beschriebene Einrichtungen können als Differenzdurchflußmesser arbeiten oder aber zusätzlich als diversitär redundantes Schutzsystem eingesetzt werden. Andererseits läßt sich auch die Verwendung dieser Systeme umkehren.

**Patentansprüche**

1. Vorrichtung zum extrakorporalen Reinigen von Blut mit Regelung der Ultrafiltrationsrate mit einem Dialysator (24), der durch eine Membran in eine erste und eine zweite Kammer geteilt ist, mit einem Dialysierflüssigkeitsweg (42), der durch die erste Kammer gelegt ist, mit einem Blutweg, der durch die zweite Kammer gelegt ist und einen ersten, zum Dialysator (24) führenden Blutleitungsteil (108) und einen zweiten, vom Dialysator abgehenden Blutleitungsteil (110) aufweist, wobei die Enden des ersten und zweiten Blutleitungsteiles (108, 110) mit einem gemeinsamen Blutanschlußteil (102) verbunden sind, mit einem ersten Absperrorgan im ersten Blutleitungsteil (108), mit einem zweiten Absperrorgan (114) im zweiten Blutleitungsteil (110), mit einer Blutpumpe (112), die im nicht betätigten Zustand das erste Absperrorgan bildet, und einem stromab zur Blutpumpe und vor dem zweiten Absperrorgan angeordneten elastischen Speicher (116), mit einer Dialysierflüssigkeitsquelle (40), von der der Dialysierflüssigkeitsweg (42) abgeht, mit einer Dialysierflüssigkeitspumpe (50) stromab des Dialysators (24) im Dialysierflüssigkeitsweg (42) sowie mit einer Steuereinheit (118), mit der alternierend in einer ersten Phase das erste Absperrorgang geöffnet und die Blutpumpe (112) betätigt sowie das zweite Absperrorgan (114) geschlossen und in einer zweiten Phase das zweite Absperrorgan (114) geöffnet und das erste Absperrorgan geschlossen wird, dadurch gekennzeichnet, daß im Blutweg eine den Durchfluß von ungereinigtem und gereinigtem Blut messende Durchflußmeßeinrichtung (104; 132, 134) vorgesehen ist, welche mit einem Ultrafiltratregler (60) verbunden ist, der einen Differenzwert zwischen den nacheinander von der Durchflußmeßeinrichtung (104; 132, 134) abgegebenen Blutflußsignalen bildet, den ermittelten Differenzwert mit einem Sollwert vergleicht und hiermit die Ultrafiltrationsrate regelt.

2. Vorrichtung nach Anspruch 1, dadurch gekennzeichnet, daß die Durchflußmeßeinrichtung (104; 132, 134) ein elektromagnetischer Durchflußmesser oder den Hämatokrit des Blutes messende Sensoren sind.

3. Vorrichtung nach Anspruch 1, dadurch gekennzeichnet, daß die Durchflußmeßeinrichtung einen einzigen Durchflußmesser (104) aufweist, der in das Blutanschlußteil (102) eingeschaltet ist.

4. Vorrichtung nach Anspruch 1, dadurch gekennzeichnet, daß die Durchflußmeßeinrichtung einen in den ersten Blutleitungsteil (108) eingeschalteten Durchflußmesser (132) aufweist und daß die Steuereinheit (118) in der ersten Phase den ersten Durchflußmesser (132) und in der zweiten Phase den zweiten Durchflußmesser (134) in Betrieb setzt.

5. Vorrichtung nach einem der Ansprüche 1 bis 4, dadurch gekennzeichnet, daß der Ultrafiltratregler (60) zur Erzeugung einer bestimmten Ultrafiltrationsrate die Drehzahl der Dialysierflüssigkeitspumpe (50) oder die zeitliche Folge der Umschaltpunkte der Steuereinheit (118) verändert.

6. Vorrichtung nach einem der Ansprüche 1 bis 5, gekennzeichnet durch einen Leitfähigkeitsmesser oder optischen Sensor als Durchflußmesser.

7. Vorrichtung nach einem der Ansprüche 1 bis 6, dadurch gekennzeichnet, daß das zweite Blutleitungsteil (110) eine Tropfkammer (30) aufweist, die über ein Leitungsstück (138), in die eine Substitutionspumpe (140) eingeschaltet ist, mit einem eine Substitutionsflüssigkeit aufweisenden Reservoir (138') verbunden ist.

8. Vorrichtung nach einem der Ansprüche 1 bis 6, dadurch gekennzeichnet, daß die Tropfkammer (30) über eine Leitung, in die ein Sterilfilter und eine weitere Pumpe eingeschaltet sind, mit dem zum Dialysator (24) führenden Leitungsabschnitt des Dialysierflüssigkeitswegs verbunden ist.

9. Vorrichtung nach einem der Ansprüche 1 bis 8, dadurch gekennzeichnet, daß im Dialysierflüssigkeitsweg Mittel zur Aufrechterhaltung eines Drucks vorgesehen sind, der unter dem im Blutweg vorliegenden Druck liegt.

10. Vorrichtung nach einem der Ansprüche 1 bis 9, dadurch gekennzeichnet, daß die Steuereinheit (118) mit einem im ersten Blutleitungsteil (108) vorgesehenen Drucksensor (120) verbunden ist und daß die Steuereinheit (118) aufgrund vorbestimmter Höchst- und Tiefstdruckwerte von der ersten in die zweite Phase und zurück schaltet und dabei jeweils über eine Leitung (130) den Ultrafiltratregler (60) startet.

## Claims

1. Apparatus for extracorporal purification of blood with controlling the ultrafiltration rate, comprising a dialyzer (24) being divided by a diaphragm into a first and a second chamber, comprising a dialysis liquid path (42) passing through the first chamber, further comprising a blood line passing through the second chamber, and a first blood line portion (108) leading to the dialyzer (24) and a second blood line portion (110) leading off the dialyzer (24), whereby the ends of the first and second blood line portion (108, 110) are connected with a common blood line connection (102), further comprising a first locking unit within the first blood line portion (108), with a second locking unit (114) within the second blood line portion (110), with a blood pump (112) forming in the non-actuated ccndition the first locking unit, and an elastic storage means (116) arranged downstream of the blood pump and before the second locking unit, with a dialysis liquid source (40) from which the dialysis liquid path (42) starts, with a dialysis liquid pump (50) downstream of the dialyzer (24) within the dialysis liquid path (42) as well as a control unit (118) by means of which alternatingly in a first phase the first locking unit is opened and the blood pump operated as well as the second locking unit (114) closed and in a second phase the second locking unit (114) is opened and the first locking unit closed, characterized in that within the blood path a flow meter unit (104; 132, 134) is provided which measures the flow of unpurified and purified blood, said flow meter being connected with an ultrafiltration control means (60) forming a difference value between the blood flow signals given subsequently by the flow meter unit (104; 132, 134), comparing the ascertained difference value with a preset point and hereby controlling the ultrafiltration rate.

2. Apparatus according to claim 1, characterized in that the flow meter unit (104; 132, 134) is an electromagnetic flow meter or sensors measuring the hematocrit of the blood.

3. Apparatus according to claim 1, characterized in that the flow meter unit comprises a single flow meter (104) which is connected into the blood line connection (102).

4. Apparatus according to claim 1, characterized in that the flow meter unit comprises a flow meter (132) which is connected into the first blood line portion (108) and that the control unit (118) in the first phase starts the first flow meter (132) and in the second phase the second flow mwter (134).

5. Apparatus according to one of claims 1 to 4, characterized in that the ultrafiltration control means (60) for generating a defined ultrafiltration rate varies the speed of the dialysis liquid pump (50) or the temporal sequence of the reverse points of the control unit (118).

6. Apparatus according to one of claims 1 to 5, characterized by an electrical conductivity meter or optical sensors as flow meters.

7. Apparatus according to one of claims 1 to 6, characterized in that the second blood line portion (110) comprises a drip chamber (30), sais drip chamber being connected via a line (138) into which a substitution pump (140) with a reservoir (138') comprising a substitution liquid is connected.

8. Apparatus according to one of claims 1 to 6, characterized in that the drip chamber (30) is connected via a line, into which a sterile filter and an additional pump are connected, with a line section of the dialysis liquid path leading to the dialyzer (24).

9. Apparatus according to one of claims 1 to 8, characterized in that in the dialysis liquid path means for maintaining a pressure are provided, said pressure being below that in the blood path.

10. Apparatus according to one of claims 1 to 9, characterized in that the control unit (118) is connected with a pressure sensor (120) provided within the first blood line portion (108) and that the control unit (118) due to the predetermined maximum- and minimum pressure values switches from the first to the second phase and vice versa thereby respectively starting the ultrafiltration control means (60) via a line (130).

## Revendications

1. Appareil pour l'épuration extracorporelle de sang avec régulation du débit d'ultrafiltration, comportant un dialyseur (24), qui est divisé par une membrane en une première et une seconde chambre, une voie de passage de liquide de dialyse (42), qui traverse la première chambre, une voie de passage de sang qui traverse la seconde chambre ainsi qu'une première partie de conduit de sang (108) aboutissant au dialyseur (24) et une seconde partie de conduit de sang (110) partant du dialyseur, les extrémités de la première et de la seconde partie de conduit de sang (108, 110 ) étant reliées à une partie commune de raccordement (102), un premier organe d'obturation placé dans la première partie de conduit de sang (108), un second organe d'obturation (114) placé dans la seconde partie de conduit de sang (110), une pompe à sang (112) qui constitue dans l'état où elle n'est pas actionnée le premier organe d'obturation, ainsi qu'un accumulateur élastique (116), disposé en aval de la pompe à sang et en amont du second organe d'obturation, une source de liquide de dialyse (40) de laquelle part la voie de passage de liquide de dialyse (42), une pompe à liquide de dialyse (50) placée en aval du dialyseur (24) dans la voie de passage de liquide de dialyse (42) ainsi qu'une unité de commande (118), au moyen de laquelle en alternance dans une première phase le premier organe d'obturation est ouvert et la pompe à sang (112) est actionnée tandis que le second organe d'obturation (114) est fermé et, dans une seconde phase, le second organe d'obturation (114) est ouvert et le premier organe d'obturation est fermé, caractérisé en ce qu'il est prévu, dans la voie d'écoulement du sang, un dispositif de mesure de débit (104; 132, 134) mesurant le débit du sang non épuré et du sang épuré et qui est relié à un régulateur d'ultrafiltrat (60), qui établit une valeur de différence entre les signaux d'écoulement sanguin produits successivement par le dispositif de mesure de débit (104; 132, 134), qui compare la la valeur de différence ainsi obtenue avec une valeur de consigne et qui règle en conséquence le débit d'ultrafiltration.

2. Appareil selon la revendication 1, caractérisé en ce que le dispositif de mesure de débit (104; 132, 134) est un débitmètre électromagnétique ou bien des capteurs mesurant l'hématocrite du sang.

3. Appareil selon la revendication 1, caractérisé en ce que le dispositif de mesure de débit comporte un seul débitmètre (104), qui est branché dans la partie de raccordement sanguin (102).

4. Appareil selon la revendication 1, caractérisé en ce que le dispositif de mesure de débit comporte un débitmètrè (132) branché dans la première partie de conduit de sang (108) et en ce que l'unité de commande (118) assure dans la première phase la mise en service du premier débitmètre (132) et dans la seconde phase la mise en service du second débitmètre (134).

5. Appareil selon une des revendications 1 à 4, caractérisé en ce que le régulateur d'ultrafiltrat (60) assure, pour produire un débit d'ultrafiltration déterminé, une modification de la vitesse de rotation de la pompe à liquide de dialyse (50) ou bien de la séquence temporelle des points de commutation de l'unité de commande (118).

6. Appareil selon une des revendications 1 à 5, caractérisé en ce qu'il utilise comme appareil de mesure de débit un appareil de mesure de conductivité ou un capteur optique.

7. Appareil selon une des revendications 1 à 6, caractérisé en ce que la seconde partie de conduit de sang (110) comporte une chambre d'égouttage (30), qui est reliée par l'intermédiaire d'un tronçon de conduit (138), dans lequel est branchée une pompe de liquide de substitution (140), avec un réservoir (138') contenant un liquide de substitution.

8. Appareil selon une des revendications 1 à 6, caractérisé en ce que la chambre d'égouttage (30) est reliée, par l'intermédiaire d'un conduit, dans lequel sont branchés un filtre stérile et une autre pompe, avec la partie de conduit, aboutissant au dialyseur (24), de la voie de passage de liquide de dialyse.

9. Appareil selon une des revendications 1 à 8, caractérisé en ce qu'il est prévu dans la voie de passage de liquide de dialyse des moyens pour maintenir une pression qui est inférieure à la pression régnant dans la voie de passage de sang.

10. Appareil selon une des revendications 1 à 9, caractérisé en ce que l'unité de commande (118) est reliée à un capteur de pression (120) disposé dans la première partie de conduit de sang (108) et en ce que l'unité de commande (118) commute, sur la base de valeurs prédéterminées de pression maximale et de pression minimale, de la première phase dans la seconde phase et en retour et assure ainsi respectivement l'enclenchement du régulateur d'ultrafiltrat (60) par l'intermédiaire d'un conduit (130).